# FASCICULE DE BREVET EUROPEEN

(11) **EP 1 879 629 B1**
(45) Date de publication et mention de la délivrance du brevet: **15.08.2012**
(21) Numéro de dépôt: 06743811.9
(22) Date de dépôt: 19.04.2006
(51) Int. Cl.: A61L 2/08

(54) **INSTALLATION POUR LA STERILISATION D'OBJETS PAR BOMBARDEMENT D'ELECTRONS**
ANORDNUNG FÜR DIE STERILISIERUNG VON GEGENSTÄNDEN DURCH ELEKTRONENBESCHUSS
ARRANGEMENT FOR STERILISING OBJECTS BY ELECTRON BOMBARDMENT

(30) Priorité: 19.04.2005 FR 0550987
(43) Date de publication de la demande: 23.01.2008
(73) Titulaire: Linac Technologies SAS, 91400 Orsay (FR)
(72) Inventeur: FONTCUBERTA, Philippe, F-41100 Vendome (FR); MORISSEAU, Didier, 91120 Palaiseau (FR)
(74) Mandataire: Poulin, Gérard
(86) Numéro de dépôt international: PCT/FR2006/050353
(87) Numéro de publication internationale: WO 2006/111681

(56) Documents cités:
- EP-A- 0 401 775
- WO-A-99/39750
- DE-A1- 3 911 749
- DE-A1- 10 235 375
- HACKETT J L: "A state of the art electron beam sterilization facility - An integrated system" RADIATION PHYSICS AND CHEMISTRY, ELSEVIER SCIENCE PUBLISHERS BV., AMSTERDAM, NL, vol. 52, no. 1-6, juin 1998 (1998-06), pages 491-494, XP004492941 ISSN: 0969-806X

## Description

### DOMAINE TECHNIQUE

La présente invention se rapporte de façon générale au domaine de la stérilisation d'objets par bombardement d'électrons, ce bombardement pouvant être du type à faible énergie dans le but de traiter la surface extérieure de ces objets, ou encore du type à énergie plus importante afin d'effectuer un traitement à coeur de ces derniers.

Elle s'applique de façon particulière mais non exclusive à la stérilisation par bombardement d'électrons d'objets de forme sensiblement parallélépipédique rectangle, tels que des objets dénommés « tubs » correspondant à des récipients fermés dans lesquels reposent une multitude d'éléments de préférence pré-stérilisés par voie chimique, comme par exemple des seringues médicales.

### ÉTAT DE LA TECHNIQUE ANTÉRIEURE

De l'art antérieur, on connaît des installations permettant d'assurer la stérilisation d'objets de forme sensiblement parallélépipédique rectangle par bombardement d'électrons à faible puissance, c'est-à-dire de puissance inférieure à environ 400 KeV.

Pour ce faire, ces installations comportent typiquement trois sources de faible énergie du type accélérateurs/canons d'environ 200 KeV, et sont disposées à 120° les unes par rapport aux autres autour d'une chambre de traitement à travers laquelle les objets sont mis en translation afin d'y être traités. De cette façon, lors de son passage en continue dans la chambre de traitement, chaque objet voit sa surface extérieure illuminée simultanément sur 360° par la combinaison des trois faisceaux d'électrons issus respectivement des sources précitées, qui ont été judicieusement positionnées.

Ce type d'installation s'est révélé satisfaisant, notamment en raison de son efficacité liée à la légère pénétration du faisceau d'électrons et à l'effet sporicide rencontrés, et également en raison de sa rapidité de traitement ainsi que de sa sécurité procurée.

Néanmoins, cette installation présente un inconvénient non négligeable résidant dans le fait qu'elle dispose d'une chambre de traitement au sein de laquelle le chemin d'objet est relativement complexe, par exemple en forme de U, afin d'assurer une protection biologique vis-à-vis des faisceaux d'électrons émis par les diverses sources des moyens de stérilisation. En effet, dans un tel cas où la chambre de traitement est délimitée par deux parois en acier inoxydable disposées de part et d'autre du chemin et s'étendant chacune tout le long du U, les moyens de stérilisation se trouvent au niveau de la base du U, tandis que les branches de celui-ci sont prévues suffisamment longues pour s'assurer que les radiations ne se propagent pas au-delà des extrémités ouvertes de ces deux branches par lesquelles les objets sont destinés à transiter. A ce titre, on peut noter que les deux extrémités ouvertes du U peuvent être assimilées à l'entrée et à la sortie de la chambre de traitement, de part et d'autre de laquelle se trouvent respectivement un premier convoyeur permettant d'amener les objets dans cette chambre, ainsi qu'un second convoyeur assurant l'évacuation des objets traités vers un isolateur de production.

Ainsi, la forme complexe de la chambre de traitement et du chemin d'objet imposée par les mesures de sécurité biologique rend l'encombrement de l'installation particulièrement important, de sorte que celle-ci n'est donc pas entièrement optimisée. De plus, il est par conséquent nécessaire de prévoir des convoyeurs courbes dans la chambre de traitement, dont la complexité et le coût sont sensiblement augmentés par rapport à des convoyeurs classiques rectilignes.

Le document EP1 340 512 A1 décrit une installation selon le préambule de la revendication 1

### EXPOSE DE L'INVENTION

L'invention a donc pour but de proposer une installation pour la stérilisation d'objets par bombardement d'électrons remédiant au moins partiellement aux inconvénients mentionnés ci-dessus relatifs aux réalisations de l'art antérieur.

Pour ce faire, l'invention a pour objet une installation pour la stérilisations d'objets par bombardement d'électrons selon la revendication 1.

On peut comprendre qu'une particularité de l'invention réside dans le fait de prévoir deux tourniquets transporteurs destinés à être mis en rotation pour déplacer les objets, et étant respectivement prévus en amont de l'entrée de la chambre de traitement, et en aval de la sortie de celle-ci. Cela procure donc une barrière pour les radiations émises par la/les sources des moyens de stérilisation, de sorte que la chambre de traitement ne nécessite plus d'être réalisée selon une forme complexe telle que celles rencontrées dans les réalisations de l'art antérieur.

L'installation pour la stérilisation d'objets peut donc avantageusement présenter un encombrement réduit, par exemple en prévoyant un chemin d'objet rectiligne à l'intérieur de la chambre de traitement, tout en procurant une protection biologique satisfaisante vis-à-vis du/des faisceaux d'électrons émis par les diverses sources des moyens de stérilisation.

Chaque tourniquet transporteur présente une pluralité de saillies radiales agencées de façon telle que deux d'entre elles quelconques et directement consécutives délimitent un logement pour recevoir un objet, ce dernier étant apte à être déplacé dans le logement lors d'une mise en mouvement du tourniquet provoquant une rotation de ce logement. Ce sont donc essentiellement ces saillies radiales, prévues par exemple au nombre de quatre, qui procurent une barrière de protection biologique vis-à-vis du/des faisceaux d'électrons, aussi bien au niveau de l'entrée que de la sortie de la chambre de traitement.

A cet égard, il est noté que l'installation comprend un premier moyen de transfert permettant de déplacer un objet situé dans un logement du tourniquet transporteur d'entrée qui communique avec une entrée de la chambre de traitement, jusqu'à l'intérieur de cette chambre de traitement. Naturellement, ce logement qui communique avec l'entrée de la chambre de traitement et dans lequel se situe un objet à traiter correspond au même logement qui était préalablement, avant la rotation du tourniquet d'entrée, agencé en regard de l'extrémité aval du premier convoyeur afin de recevoir l'objet provenant de celui-ci.

Par ailleurs, l'installation comprend un deuxième moyen de transfert permettant de déplacer un objet situé à l'intérieur de la chambre de traitement, jusque dans un logement du tourniquet transporteur de sortie qui communique avec une sortie de cette chambre de traitement. Par conséquent, une fois que l'objet est situé à l'intérieur du logement qui vient d'être mentionné, le tourniquet transporteur de sortie peut être mis en rotation afin de déplacer cet objet de sorte qu'il soit entièrement extrait de la chambre de traitement. De manière préférée, l'installation comprend un troisième moyen de transfert permettant de déplacer un objet situé à une extrémité aval du premier convoyeur, jusque dans un logement du tourniquet transporteur d'entrée agencé en regard de cette extrémité aval du premier convoyeur. Ainsi, une fois que l'objet est situé à l'intérieur du logement précité, le tourniquet d'entrée peut être mis en rotation afin de déplacer cet objet dans une position lui permettant d'être introduit dans la chambre de traitement.

A cet égard, il est noté que l'installation comprend un quatrième moyen de transfert permettant de déplacer un objet situé dans un logement du tourniquet transporteur de sortie agencé en regard d'une extrémité amont du second convoyeur, jusque sur cette extrémité amont du second convoyeur. Ici encore, il est à comprendre que logement agencé en regard de l'extrémité amont du second convoyeur correspond au même logement, qui, avant la rotation du tourniquet de sortie, était en communication avec la sortie de la chambre de traitement.

La chambre de traitement est équipée d'au moins un convoyeur interne permettant de mettre les objets en mouvement à l'intérieur de cette chambre, et de préférence équipée d'un convoyeur interne primaire sur lequel les objets peuvent être amenés par le second moyen de transfert, et d'un convoyeur interne secondaire depuis lequel les objets peuvent être déplacés dans un logement du tourniquet transporteur de sortie, par le deuxième moyen de transfert.

Dans cette configuration particulièrement adaptée aux cas où il est mis en oeuvre une pluralité de sources émettant chacune un faisceau d'électrons, on peut prévoir que le convoyeur interne primaire dispose d'une extrémité amont située à proximité de l'entrée de la chambre de traitement, et que le convoyeur interne secondaire dispose d'une extrémité aval située à proximité de la sortie de la chambre de traitement.

De plus, il est également prévu que l'extrémité aval du convoyeur interne primaire est située en regard et à distance d'une extrémité amont du convoyeur interne secondaire, de manière à former un espace inter-convoyeur susceptible d'être traversé par les faisceaux d'électrons des moyens de stérilisation.

De préférence, les premier convoyeur, second convoyeur, convoyeur interne primaire et convoyeur interne secondaire sont chacun sensiblement rectilignes, et disposés sensiblement selon une même ligne droite parallèle à une direction d'avancement des objets. Ainsi, le chemin d'objet à l'intérieur de la chambre de traitement est rectiligne, ce qui procure un encombrement relativement faible. De plus, si il a été préférentiellement prévu d'aligner également les premier et second convoyeurs disposés de part et d'autre de cette chambre, ceux-ci peuvent néanmoins être disposés autrement, sans sortir du cadre de l'invention. A titre d'exemple illustratif, le premier convoyeur pourrait être disposé à 90° par rapport au convoyeur interne primaire, de même que le second convoyeur pourrait aussi être disposé à 90° par rapport au convoyeur interne secondaire. Cette faculté de modifier la disposition des premier et second convoyeurs est naturellement procurée par la présence des tourniquets transporteurs d'entrée et de sortie, pour lesquels il est uniquement nécessaire de modifier l'étendue des rotations pour pouvoir s'adapter aux diverses dispositions des convoyeurs.

Comme évoqué précédemment, les moyens de stérilisation comprennent une pluralité de sources générant chacune un faisceau d'électrons selon un axe traversant la chambre de traitement, et de préférence trois sources générant respectivement trois faisceaux d'électrons selon des axes respectivement disposés à 120° les uns par rapport aux autres, dans un plan P orthogonal à une direction d'avancement des objets.

Dans cette solution préférée particulièrement adaptée pour assurer un traitement dit « de surface », la stérilisation consiste à réaliser un bombardement d'électrons à faible énergie sur la surface des objets. Ainsi, lors de son passage en continue dans la chambre de traitement, au niveau de l'espace inter-convoyeur, chaque objet voit sa surface extérieure illuminée simultanément sur 360° par la combinaison des trois faisceaux d'électrons issus respectivement des sources précitées, qui ont été judicieusement positionnées. A cet égard, il est bien évidemment précisé que cela est possible en prévoyant que le plan P traverse l'espace inter-convoyeur.

Toujours de manière préférentielle, les sources sont agencées de manière à ce que chacun des trois axes traverse un objet à traiter placé au niveau de cet espace inter-convoyeur.

Naturellement, cette solution à trois sources ou plus est également envisageable pour assurer un traitement dit « à coeur », selon lequel la stérilisation consiste à réaliser un bombardement d'électrons à plus forte énergie sur les objets, la puissance pouvant alors être supérieure à 400 KeV, et atteindre 5 voire 10 MeV. Naturellement, pour le traitement à coeur, le nombre de sources peut être diminué ou augmenté en fonction des besoins rencontrés, et ne se limite donc pas à trois.

De préférence, le second convoyeur débouche dans un isolateur de production dans lequel sont acheminés les objets stérilisés.

Il est noté que l'invention permet aussi de d'effectuer un traitement à coeur d'objets conditionnés dans leur emballage final. Dans un tel cas, le second convoyeur débouche dans un isolateur de production, ou dans un autre environnement protégé, car l'emballage lui-même protège le produit après traitement.

De manière préférée, le tourniquet transporteur d'entrée est placé dans une couronne présentant d'une part une ouverture en regard du premier convoyeur pour pouvoir autoriser l'introduction des objets dans le tourniquet transporteur d'entrée, et d'autre part une ouverture située dans le prolongement et en amont de l'entrée de la chambre de traitement. De façon similaire, le tourniquet transporteur de sortie est placé dans une couronne présentant d'une part une ouverture située dans le prolongement et en aval de la sortie de la chambre de traitement, et d'autre part une ouverture située en regard du second convoyeur pour pouvoir autoriser l'extraction des objets du tourniquet transporteur de sortie.

Il est noté que ces deux couronnes servent chacune simultanément à procurer une barrière pour les radiations émises par les faisceaux, et à éviter l'extraction des objets situés dans les logements des tourniquets transporteurs, lors de la mise en rotation de ces derniers.

Enfin, comme cela a été évoqué ci-dessus, il est noté que l'installation est réalisée de manière à permettre un traitement de surface ou à coeur des objets à traiter.

D'autres avantages et caractéristiques de l'invention apparaîtront dans la description détaillée non limitative ci-dessous.

### BRÈVE DESCRIPTION DES DESSINS

Cette description sera faite au regard des dessins annexés parmi lesquels ;
- la figure 1 représente une vue de face d'une installation pour la stérilisation d'objets par bombardement d'électrons, selon un mode de réalisation préféré de la présente invention ;
- la figure 2 représente une vue partielle en perspective de l'installation montrée sur la figure 1, les objets à traiter ayant été volontairement omis de la représentation pour des raisons de clarté ;
- la figure 3 représente une vue schématique de dessus de l'installation montrée sur les figures 1 et 2, destinée à montrer le chemin des objets au sein de cette installation ;
- la figure 4 représente une vue schématique de face de l'installation montrée sur les figures 1 et 2, destinée à montrer les divers moyens de mis en mouvement des objets présents au sein de cette installation ; et
- la figure 5 représente une vue partielle en coupe prise le long de la ligne V-V de la figure 1, destinée à montrer plus spécifiquement les moyens de stérilisation de l'installation.

### EXPOSÉ DÉTAILLÉ DE MODES DE RÉALISATION PRÉFÉRÉS

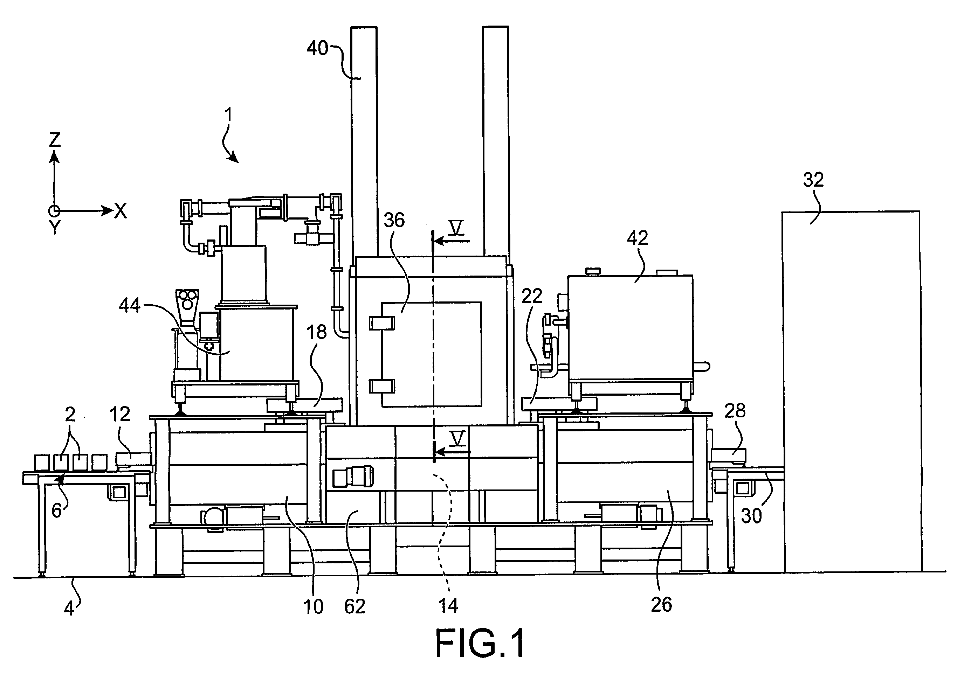

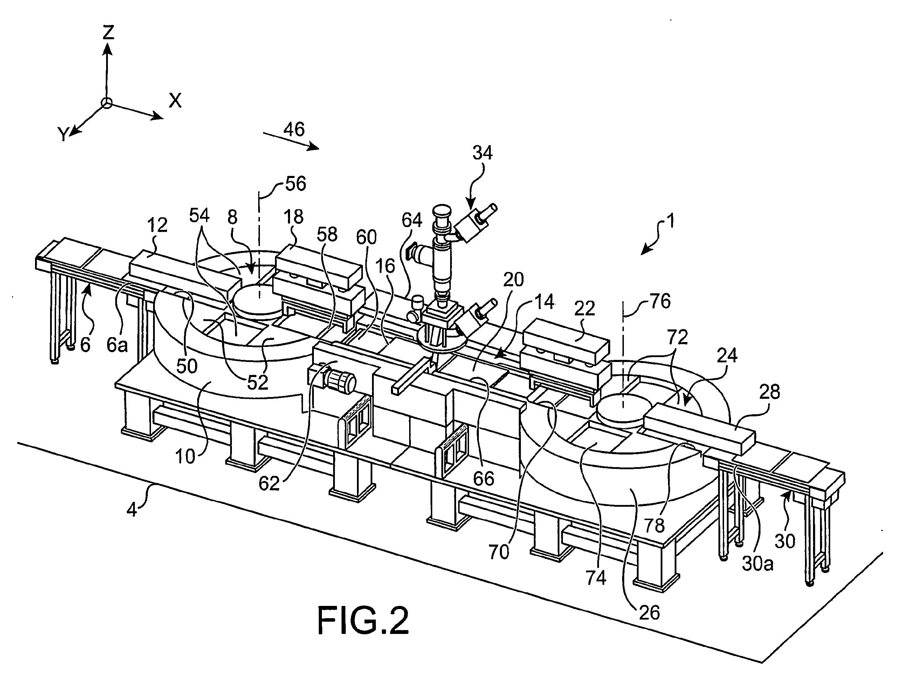

En référence tout d'abord conjointement aux figures 1 et 2, on peut apercevoir une installation 1 pour la stérilisation d'objets 2 par bombardement d'électrons, cette installation 1 étant préférentiellement mais non exclusivement destinée au traitement d'objets de forme sensiblement parallélépipédique rectangle. De plus, même si l'application décrite ci-après concerne le traitement de surface des objets à stériliser, elle peut naturellement s'appliquer pour un traitement à coeur de ces derniers.

Comme cela a été évoqué précédemment, cette forme d'objet correspond notamment à celle de tubs dans lesquels reposent une multitude d'éléments ayant été pré-stérilisés par voie chimique, tels que des seringues médicales pouvant par exemple être au nombre de cent par tub.

L'installation 1 est posée sur un sol 4, que l'on peut assimiler à un plan horizontal. A ce titre, on peut noter que la description sera faite en référence à une direction X parallèle au sol 4 et correspondant à une direction d'avancement des objets au sein de l'installation, une direction Y correspondant à une direction transverse de l'installation également parallèle au sol 4, ainsi qu'à une direction Z correspondant à une direction de la hauteur qui est quant à elle orthogonale à ce même sol 4, les directions X, Y et Z étant orthogonales entre elles.

Globalement, l'installation comporte les éléments suivants : un premier convoyeur 6, un tourniquet transporteur d'entrée 8 placé dans une couronne 10, un troisième moyen de transfert d'objet 12, une chambre de traitement 14 dans laquelle se situent un convoyeur interne primaire 16 coopérant avec un premier moyen de transfert d'objet 18 ainsi qu'un convoyeur interne secondaire 20 coopérant avec un second moyen de transfert d'objet 22, un tourniquet transporteur de sortie 24 placé dans une couronne 26, un quatrième moyen de transfert 28, et un second convoyeur 30 débouchant dans un isolateur de production 32.

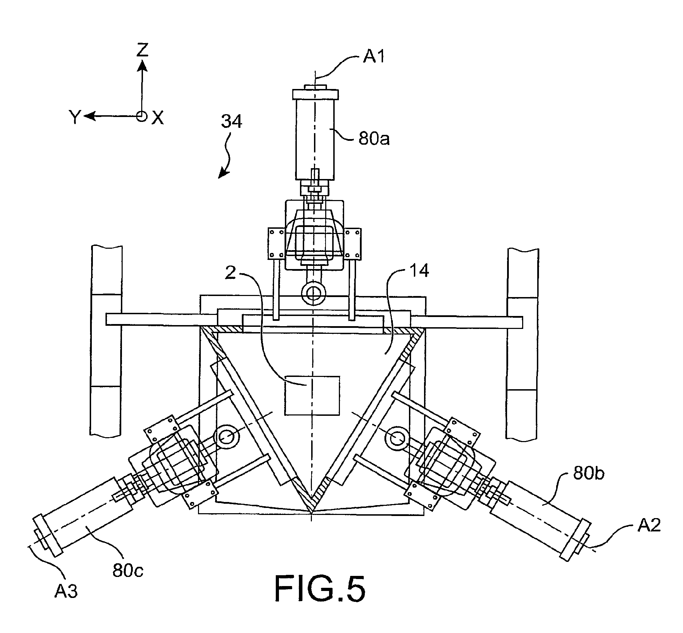

D'autre part, cette installation 1 comprend également des moyens de stérilisation 34 qui seront décrits de façon détaillée ultérieurement en référence à la figure 5, et qui ont la particularité d'une part de produire au moins un faisceau d'électrons au sein de la chambre de traitement 14, et d'autre part d'être enfermés dans un coffrage blindé 36 tel que montré sur la figure 1. A titre indicatif, le coffrage blindé 36 peut être réalisé en plomb sur une épaisseur d'environ 15 mm dans le cadre d'un traitement à basse énergie (environ 200 KeV), ou d'environ 600 mm dans le cadre d'un traitement à énergie plus élevée (de l'ordre par exemple de 5 MeV).

L'installation 1 présente également deux conduits 38, 40 communiquant avec la chambre de traitement 14 et servant respectivement à l'amenée d'air frais, et à l'extraction de l'ozone produite par le bombardement d'électrons.

D'autre part, des moyens de refroidissement 42 ainsi qu'un klystron 44 relié aux moyens de stérilisation 34 équipent également l'installation 1.

Plus particulièrement en référence à la figure 2, il est représenté une direction d'avancement des objets 46 au sein de l'installation 1, qui est parallèle à la direction X. Les quatre convoyeurs 6, 16, 20, 30 sont préférentiellement droits/rectilignes et alignés selon cette direction 46, comme cela est clairement visible sur cette figure 2.

Le premier convoyeur 6 est un convoyeur gravitaire à rouleaux (ou d'un autre type approprié) assurant un déplacement automatique des objets 2 à traiter, sensiblement selon la direction X. Il dispose d'une extrémité aval 6a située en amont et en regard d'une ouverture 50 pratiquée dans la couronne 10 pour laisser passer les objets 2, et permettre l'introduction de ces derniers dans le transporteur d'entrée 8. A cet égard, au même titre que pour certains des autres éléments de l'installation 1 qui seront décrits ci-après, cette couronne 10 est de préférence et principalement réalisée en plomb dans le but essentiellement d'assurer une protection biologique totale vis-à-vis des faisceaux d'électrons émis par les moyens de stérilisation 34 durant le traitement.

A l'intérieur de cette couronne 10 se situe le tourniquet transporteur d'entrée 8 qui présente une pluralité de saillies radiales 52, de préférence quatre et principalement en plomb, agencées de façon telle que deux d'entre elles quelconques et directement consécutives délimitent un logement 54 pour recevoir un objet 2. Comme on peut le voir sur la figure 2, les saillies 52 délimitant quatre logements 54 répartis régulièrement peuvent disposer d'une forme permettant de définir deux à deux des logements 54 sensiblement parallélépipédiques, de forme complémentaire de celle des objets 2, ou de celle d'un assemblage d'objets dans le cadre d'un traitement à coeur. Ainsi, chaque objet 2 peut alors être mis en rotation dans l'un des logements 54 selon un axe 56 correspondant à l'axe de rotation du tourniquet 8, lors d'une mise en mouvement de ce même tourniquet. Il est noté à titre indicatif que l'axe vertical 56, correspondant également à l'axe de la couronne 10 dont la surface intérieure est épousée par les extrémités libres des saillies radiales 52, est de préférence situé dans un plan vertical parallèle à la direction 46 et traversant de façon fictive les quatre convoyeurs alignés, de préférence en leur milieu.

Au niveau d'une partie aval de la couronne 10, celle-ci présente une autre ouverture 58 qui se situe dans le prolongement et en amont d'une entrée 60 de la chambre de traitement 14, cette ouverture 58 étant diamétralement opposée à l'ouverture 50.

La chambre de traitement 14 est délimitée par deux parois 62, 64 principalement en plomb, qui sont orientées selon des plans XZ et qui définissent conjointement l'entrée 60 ainsi que la sortie 66 de cette chambre. Entre ces deux parois 62, 64 assurant la protection biologique, se situent les deux convoyeurs internes 16, 20, dont une extrémité amont 16a du convoyeur interne primaire 16 est située à proximité de l'entrée 60, et dont une extrémité aval 20a du convoyeur interne secondaire 20 est agencée à proximité de la sortie 66.

En outre, l'extrémité aval 16b du convoyeur 16 et l'extrémité amont 20b du convoyeur 20 sont situées en regard l'une de l'autre, et sont séparées par un espace inter-convoyeur 68 dont la longueur selon la direction X est fixée de manière à être inférieure à la longueur dans cette même direction d'un objet 2 à traiter posé sur le convoyeur interne 16, de façon à ce que l'objet en question puisse transiter automatiquement d'un convoyeur interne à l'autre. L'effet recherché est en réalité de pouvoir obtenir une continuité d'entraînement d'un objet 2 d'un bout à l'autre de la chambre de traitement 14 et uniquement à l'aide des deux convoyeurs internes 16, 20 à vitesse régulée, tout en offrant l'espace inter-convoyeur 68 pour permettre aux faisceaux d'électrons de pouvoir illuminer la surface extérieure de cet objet 2 sur simultanément 360° lorsqu'il passe au-dessus de cet espace 68.

Dans le prolongement de la sortie 66 délimitée par les deux parois parallèles 62, 64, se trouve une ouverture 70 de la couronne 26 dans laquelle se situe le tourniquet transporteur de sortie 24, l'agencement entre ce dernier et la couronne 26 réalisée principalement en plomb étant sensiblement identique à celui décrit entre la couronne 10 et le tourniquet d'entrée 8.

Plus précisément, le tourniquet de sortie 24 présente une pluralité de saillies radiales 72, de préférence quatre et principalement en plomb, agencées de façon telle que deux d'entre elles quelconques et directement consécutives délimitent un logement 74 pour recevoir un objet 2. Comme on peut le voir sur la figure 2, les saillies 72 délimitant quatre logements 74 répartis régulièrement peuvent disposer d'une forme permettant de définir deux à deux des logements 74 sensiblement parallélépipédiques, de forme complémentaire de celle des objets 2. Ainsi, chaque objet 2 peut alors être mis en rotation dans l'un des logements 74 selon un axe 76 correspondant à l'axe de rotation du tourniquet 24, lors d'une mise en mouvement de ce même tourniquet. Il est noté à titre indicatif que l'axe vertical 76, correspondant également à l'axe de la couronne 26 dont la surface intérieure est épousée par les extrémités libres des saillies radiales 72, est de préférence situé dans le plan vertical traversant de façon fictive les quatre convoyeurs alignés.

Au niveau d'une partie aval de la couronne 24, celle-ci présente une autre ouverture 78 diamétralement opposée à l'ouverture 70, permettant le passage des objets 2 à travers cette couronne 24, et étant située en amont et en regard d'une extrémité amont 30a du second convoyeur 30 menant ces objets 2 vers l'isolateur de production 32. Cette ouverture 78 a principalement pour fonction d'autoriser l'extraction des objets 2 hors du tourniquet de sortie 24.

Le second convoyeur 30 est également un convoyeur gravitaire à rouleaux (ou d'un autre type approprié) assurant un déplacement automatique des objets 2 à traiter, sensiblement selon la direction X.

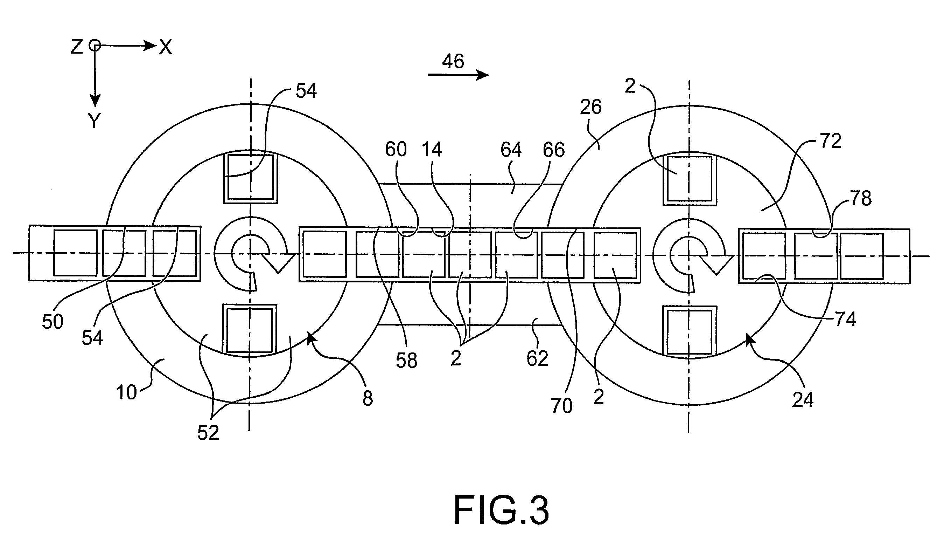

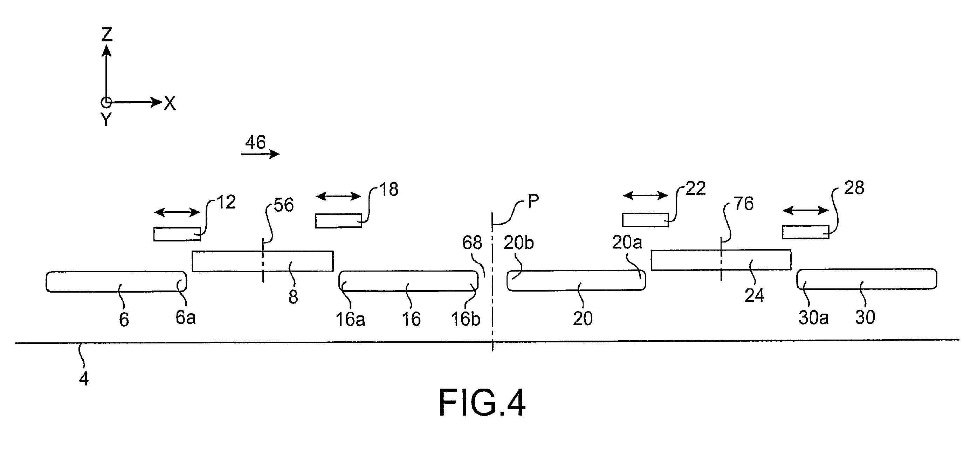

En référence à présent plus spécifiquement aux figures 3 et 4, il va être décrit le chemin d'un objet 2 donné à travers l'installation 1.

Tout d'abord, le tourniquet 8 est mis en rotation autour de l'axe 56 de manière à ce que l'un de ses logements 54 soit placé en regard de l'ouverture 50. Ensuite, le troisième moyen de transfert 12 est piloté de manière à déplacer dans la direction X l'objet 2 situé le plus en aval sur le premier convoyeur 6, jusque dans le logement 54 précité, en passant par l'ouverture 50.

Le tourniquet d'entrée 8 effectue alors deux quarts de tour avant de présenter cet objet 2 en regard de l'autre ouverture 58. Le premier moyen de transfert 18 est piloté de manière à déplacer dans la direction X l'objet 2 jusqu'à l'intérieur de la chambre de traitement 14, en passant par l'ouverture 58 et l'entrée 60. Il est bien entendu qu'à chaque pas du tourniquet 8, un objet 2 est entré tandis qu'un autre objet 2 est sorti, afin d'optimiser la cadence.

Une fois que celui-ci a pénétré dans cette chambre 14, le déplacement de l'objet 2 dans la direction X est automatiquement assuré tout d'abord par le convoyeur interne d'entrée 16, puis par le convoyeur interne de sortie 20, ces deux convoyeurs étant à vitesse régulée. A cet égard, il est noté que c'est lorsque l'objet 2 passe au-dessus de l'espace inter-convoyeur 68, c'est-à-dire lorsqu'il repose simultanément sur l'extrémité aval 16b et l'extrémité amont 20b des convoyeurs 16, 20, qu'il subit le traitement le plus intense, puisqu'il est alors illuminé sur 360° par les trois faisceaux d'électrons qui seront décrits ci-après.

Le déplacement de l'objet 2 sur le convoyeur 20 à vitesse régulée est opéré jusqu'à ce que celui-ci atteigne la sortie 66, au niveau de laquelle cet objet 2 est en mesure de coopérer avec le troisième moyen de transfert 22 piloté de manière à le déplacer dans la direction X, jusqu'à l'intérieur d'un logement 74 du tourniquet de sortie 24, en passant par la sortie 66 et l'ouverture 70 de la couronne 26. Pour ce faire, le tourniquet 24 est préalablement mis en rotation autour de son axe 76 de manière à ce que l'un de ses logements 74 soit placé en regard de l'ouverture 70.

Lorsque l'objet 2 est situé dans le logement 74, le tourniquet de sortie 24 effectue alors deux quarts de tour avant de présenter cet objet 2 en regard de l'autre ouverture 78. Le quatrième moyen de transfert 28 est alors piloté de manière à déplacer dans la direction X l'objet 2 jusqu'à ce qu'il atteigne l'extrémité amont 30a du second convoyeur 30, en passant par l'ouverture 78. Ici encore, il est bien entendu qu'à chaque pas du tourniquet 24, un objet 2 est entré tandis qu'un autre objet 2 est sorti, afin d'optimiser la cadence.

Enfin, l'objet 2 est ensuite déplacé automatiquement dans la direction X par gravité sur le convoyeur 30, jusque dans l'isolateur de production 32.

Naturellement, comme cela est représenté sur la figure 3, il est noté que l'installation 1 est conçue pour pouvoir accueillir plusieurs objets 2 simultanément, impliquant que plusieurs d'entre eux sont donc agencés les uns derrière les autres à l'intérieur de la chambre de traitement 14. Une autre conséquence réside bien entendu dans le fait qu'en fonctionnement, à chaque quart de tour (à chaque pas) du tourniquet transporteur d'entrée 8, un nouvel objet 2 se présente en regard de l'entrée 60 de la chambre de traitement 14, avant d'y pénétrer par l'intermédiaire du premier moyen de transfert 18.

A titre indicatif, les tourniquets 8, 24 peuvent être associés à des moteurs électriques classiques pour leur mise en rotation, tandis que les éléments 12, 18, 22, 28 peuvent chacun prendre la forme d'un chariot en acier inoxydable monté sur vis ou bille, et actionné par un moteur électrique classique. D'autre part, les éléments 16 et 20 peuvent quant à eux chacun prendre la forme d'une bande transporteuse en maille en acier inoxydable, et actionnée par un moteur électrique classique.

En référence à présent à la figure 5 représentant les moyens de stérilisation 34 se présentant sous une forme préférée particulièrement adaptée à un traitement de surface des objets 2, on peut voir que ceux-ci présentent trois sources 80a, 80b, 80c générant chacune un faisceau d'électrons selon des axes A1, A2, A3, respectivement.

Ces axes sont globalement disposés à 120° les uns par rapport aux autres dans un plan P représenté sur la figure 4 et correspondant à la ligne V-V sur la figure 1. Ce plan P est orthogonal à la direction d'avancement des objets 46, et traverse l'espace inter-convoyeur 68 préférentiellement en son milieu. C'est la raison pour laquelle un objet 2 passant au-dessus de cet espace inter-convoyeur 68 voit sa surface extérieure illuminée sur 360°, par l'action conjointe des trois sources 80a, 80b, 80c dont les axes A1, A2, A3 traversent de préférence chacun cet objet 2 en mouvement au-dessus de l'espace 68.

## Revendications

1. Installation (1) pour la stérilisation d'objets par bombardement d'électrons, comprenant des moyens de stérilisation (34) aptes à générer au moins un faisceau d'électrons selon un axe (A1, A2, A3) traversant une chambre de traitement (14) de l'installation au sein de laquelle les objets (2) sont destinés à être mis en mouvement, ladite installation comprenant un premier convoyeur (6) ainsi qu'un second convoyeur (30) conçus pour déplacer lesdits objets (2) et étant respectivement situés de part et d'autre de ladite chambre de traitement (14), ladite installation étant munie d'une part d'un tourniquet transporteur d'entrée (8) conçu pour déplacer lesdits objets (2) et étant interposé entre le premier convoyeur (6) et ladite chambre de traitement (14), et d'autre part d'un tourniquet transporteur de sortie (24) également conçu pour déplacer lesdits objets (2) et étant interposé entre ladite chambre de traitement (14) et le second convoyeur (30), lesdits tourniquets transporteurs (8, 24) étant conçus et disposés de façon à assurer une barrière aux radiations émises par lesdits moyens de stérilisation (34),
chaque tourniquet transporteur (8, 24) présentant une pluralité de saillies radiales (52, 72) agencées de façon telle que deux d'entre elles quelconques et directement consécutives délimitent un logement (54, 74) pour recevoir un objet (2), ce dernier étant apte à être déplacé dans ledit logement (54, 74) lors d'une mise en mouvement du tourniquet (8, 24) provoquant une rotation de ce logement ;
**caracterisée en ce qu'**elle comprend un premier moyen de transfert (18) permettant de déplacer un objet (2) situé dans un logement (54) du tourniquet transporteur d'entrée (8) qui communique avec une entrée (60) de ladite chambre de traitement (14), jusqu'à l'intérieur de cette chambre de traitement (14) ;
**en ce qu'**elle comprend un deuxième moyen de transfert (22) permettant de déplacer un objet (2) situé à l'intérieur de ladite chambre de traitement (14), jusque dans un logement (74) du tourniquet transporteur de sortie (24) qui communique avec une sortie (66) de cette chambre de traitement (14) ;
**en ce que** ladite chambre de traitement (14) est équipée d'au moins un convoyeur interne (16, 20) permettant de mettre les objets (2) en mouvement à l'intérieur de cette chambre (14), parmi lesquels un convoyeur interne primaire (16) sur lequel les objets (2) peuvent être amenés par ledit second moyen de transfert (18), et un convoyeur interne secondaire (20) depuis lequel les objets (2) peuvent être déplacés dans un logement (74) du tourniquet transporteur de sortie (24), par ledit deuxième moyen de transfert (28) ;
**en ce qu'**une extrémité aval (16b) du convoyeur interne primaire (16) est située en regard et à distance d'une extrémité amont (20b) du convoyeur interne secondaire (20), de manière à former un espace inter-convoyeur (68) ; et
**en ce que** lesdits moyens de stérilisation (34) comprennent une pluralité de sources (80a, 80b, 80c) générant chacune un faisceau d'électrons selon un axe (A1, A2, A3) traversant chacun ledit espace inter-convoyeur (68).

2. Installation (1) pour la stérilisation d'objets selon la revendication 1, **caractérisée en ce qu'**elle comprend un troisième moyen de transfert (12) permettant de déplacer un objet (2) situé à une extrémité aval (6a) du premier convoyeur (6), jusque dans un logement (54) du tourniquet transporteur d'entrée (8) agencé en regard de cette extrémité aval (6a) du premier convoyeur (6).

3. Installation (1) pour la stérilisation d'objets selon la revendication 1 ou la revendication 2, **caractérisée en ce qu'**elle comprend un quatrième moyen de transfert (28) permettant de déplacer un objet (2) situé dans un logement (74) du tourniquet transporteur de sortie (24) agencé en regard d'une extrémité amont (30a) du second convoyeur (30), jusque sur cette extrémité amont du second convoyeur (30).

4. Installation (1) pour la stérilisation d'objets selon l'une quelconque des revendications précédentes, **caractérisée en ce que** ledit convoyeur interne primaire (16) dispose d'une extrémité amont (16a) située à proximité de l'entrée (60) de ladite chambre de traitement (14), et **en ce que** ledit convoyeur interne secondaire (20) dispose d'une extrémité aval (20a) située à proximité de la sortie (66) de ladite chambre de traitement (14).

5. Installation (1) pour la stérilisation d'objets selon l'une quelconque des revendications précédentes, **caractérisée en ce que** lesdits convoyeur interne primaire (16) et convoyeur interne secondaire (20) sont chacun sensiblement rectilignes, et disposés sensiblement selon une même ligne droite parallèle à une direction d'avancement des objets (46).

6. Installation (1) pour la stérilisation d'objets selon l'une quelconque des revendications précédentes, **caractérisée en ce que** lesdites moyens de stérilisation (34) comprennent trois sources (80a, 80b, 80c) générant respectivement trois faisceaux d'électrons selon des axes (A1, A2, A3) traversant ladite chambre de traitement (14), lesdits axes (A1, A2, A3) étant respectivement disposés à 120° les uns par rapport aux autres dans un plan P orthogonal à une direction d'avancement des objets (46).

7. Installation (1) pour la stérilisation d'objets selon la revendication 6, **caractérisée en ce que** ledit plan P traverse ledit espace inter-convoyeur (68).

8. Installation (1) pour la stérilisation d'objets selon l'une quelconque des revendications précédentes, **caractérisée en ce que** ledit second convoyeur (30) débouche dans un isolateur de production (32) dans lequel sont acheminés les objets stérilisés (2).

9. Installation (1) pour la stérilisation d'objets selon l'une quelconque des revendications précédentes, **caractérisée en ce que** ledit tourniquet transporteur d'entrée (8) est placé dans une couronne (10) présentant d'une part une ouverture (50) en regard du premier convoyeur (6) pour pouvoir autoriser l'introduction des objets (2) dans ledit tourniquet transporteur d'entrée (8), et d'autre part une ouverture (58) située dans le prolongement et en amont de l'entrée (60) de ladite chambre de traitement (14), et **en ce que** ledit tourniquet transporteur de sortie (24) est placé dans une couronne (26) présentant d'une part une ouverture (70) située dans le prolongement et en aval de la sortie (66) de ladite chambre de traitement (14), et d'autre part une ouverture (78) située en regard du second convoyeur (30) pour pouvoir autoriser l'extraction des objets (2) dudit tourniquet transporteur de sortie (24).

## Claims

1. A facility (1) for sterilizing objects by electron bombardment, comprising sterilization means (34) able to generate at least one electron beam along an axis (A1, A2, A3) passing through a treatment chamber (14) of the facility within which said objects (2) are intended to be set into motion, said facility comprising a first conveyor (6) as well as a second conveyor (30) used for moving said objects (2) and being respectively located on either side of said treatment chamber (14), **characterized in that** said facility is provided with an input rotatable conveyor (8) designed for moving said objects (2) and placed between the first conveyor (6) and said treatment chamber (14) on the one hand, and an output rotatable conveyor (24) also designed for moving said objects (2) and placed between said treatment chamber (14) and the second conveyor (30), said rotatable conveyors (8, 24) each being designed and placed so as to provide a barrier against the radiations emitted by said sterilization means (34),
each rotatable conveyor (8, 24) having a plurality of radial protections (52, 72) laid out such that any two of them being directly consecutive to each other define a housing (54, 74) to receive an object (2), the latter being able to be moved in said housing (54, 74) when the rotatable conveyor (8, 24) is set into motion, causing rotation of this housing;
**characterized in that** it comprises a first transfer means (18) which make it possible to move an object (2) located in a housing (54) of the input rotatable conveyor (8) which communicates with an inlet (60) of said treatment chamber (14), right up into the inside of this treatment chamber (14);
**in that** it comprises a second transfer means (22) which make it possible to move an object (2) located inside said treatment chamber (14), right up into a housing (74) of the output rotatable conveyor (24) which communicates with an outlet (66) of this treatment chamber (14);
**in that** said treatment chamber (14) is provided with at least one internal conveyor (16, 20) which makes it possible to set the objects (2) into motion inside this chamber (14), including a primary internal conveyor (16) onto which objects (2) may be brought by said second transfer means (18), and a secondary internal conveyor (20) from which objects (2) may be moved into a housing (74) of the output rotatable conveyor (24), by said second transfer means (28);
**in that** a downstream end (16b) of the primary internal conveyor (16) is located facing and away from an upstream end (20b) of the secondary internal conveyor (20), so as to form an inter-conveyor space (68); and
**in that** said sterilization means (34) comprise a plurality of sources (80a, 80b, 80c) each generating an electron beam along an axis (A1, A2, A3) passing through said inter-conveyor space (68).

2. The facility (1) for sterilizing objects according to claim 1, **characterized in that** it comprises a third transfer means (12) which make it possible to move an object (2) located at a downstream end (6a) of the first conveyor (6), into a housing (54) of the input rotatable conveyor (8) laid out facing this downstream end (6a) of the first conveyor (6).

3. The facility (1) four sterilizing objects according to claim 1 or claim 2, **characterized in that** it comprises a fourth transfer means (28) which make it possible to move an object (2) located in a housing (74) of the output rotatable conveyor (24) laid out facing an upstream end (30a) of the second conveyor (30), right up onto this upstream end of the second conveyor (30).

4. The facility (1) for sterilizing objects according to any of the preceding claims, **characterized in that** said primary internal conveyor (16) has an upstream end (16a) located in proximity to the inlet (60) of said processing chamber (14), and **in that** said secondary internal conveyor (20) has a downstream end (20a) located in proximity to the outlet (66) of said treatment chamber (19).

5. The facility (1) for sterilizing objects according to any of the preceding claims, **characterized in that** said primary internal conveyor (16) and secondary internal conveyor (20) are each substantially rectilinear, and substantially positioned along a same straight line parallel to a direction of forward motion of the objects (46).

6. The facility (1) for sterilizing objects according to any of the preceding claims, **characterized in that** said sterilization means (34) comprise three sources (80a, 80b, 80c) respectively generating three electron beams along axes (A1, A2, A3) passing through said treatment chamber (14), said axes (A1, A2, A3) being respectively positioned at 120° relative to each other in a plane P orthogonal to a direction of forward motion of the objects (96).

7. The facility (1) for sterilizing objects according to claim 6, **characterized in that** said plane P passes through said inter-conveyor space (68).

8. The facility (1) for sterilizing objects according to any of the preceding claims, **characterized in that** said second conveyor (30) opens into a production isolator (32) into which the sterilized objects (2) are brought.

9. The facility (1) for sterilizing objects according to any of the preceding claims, **characterized in that** said input rotatable conveyor (8) is placed in a crown (10) having an opening (50) facing the first conveyor (6) ro allow the introduction of objects (2) into said input rotatable conveyor (8), and an opening (58) located in the extension of the inlet (60) of said treatment chamber (14), and upstream therefrom and **in that** said output rotatable conveyor (24) is placed in a crown (26) having an opening (70) located in the extension of the outlet (66) of said treatment chamber (14) and downstream therefrom and an opening (78) located facing the second conveyor (30) to allow extraction of the objects (2) from said output rotatable conveyor (24).

## Patentansprüche

1. Anordnung (1) für die Sterilisierung von Gegenständen mittels Elektronenbeschuss, die Anordnung umfassend Sterilisierungsmittel (34), die wenigstens ein Elektronenstrahlbündel entlang einer Achse (A1, A2, A3) erzeugen können, welche eine Behandlungskammer (14) der Anordnung durchsetzt, in deren Innerem die Gegenstände (2) bestimmungsgemäß in Bewegung versetzt werden, die Anordnung des weiteren umfassend einen ersten (3) sowie einen zweiten (30) Förderer bzw. Konveyer, die zur Verschiebung der genannten Gegenstände (2) bestimmt sind und jeweils beidseits der genannten Behandlungskammer (14) angeordnet sind, wobei die Anordnung einerseits mit einem Eingangs-Transport-Drehkreuz bzw. -Drehteller (8), welcher zur Verschiebung bzw. zum Transport der genannten Gegenstände (2) bestimmt ist und zwischen dem ersten Förderer bzw. Konveyer (6) und der genannten Behandlungskammer (14) angeordnet ist, und andererseits mit einem Ausgangs-Transport- Drehkreuz bzw. -Drehteller (24) versehen ist, das bzw. der ebenfalls zur Verschiebung bzw. zum Transport der genannten Gegenstände (2) bestimmt ist und zwischen der genannten Behandlungskammer (14) und dem zweiteten Förderer bzw. Konveyer (30) angeordnet ist, wobei die genannten Drehkreuze bzw. Drehteller (8, 24) dazu bestimmt und so angeordnet sind, eine Barriere für die von den genannten Sterilisierungsmitteln (34) ausgehenden Strahlungen zu gewährleisten, und wobei jeweils jedes Drehkreuz bzw. Drehteller (8, 24) eine Mehrzahl von radialen Vorsprüngen bzw. Ansätzen (52, 74) solcher Ausbildung und Anordnung aufweist, dass jeweils zwei beliebige und unmittelbar aufeinanderfolgende von diesen Vorsprüngen bzw. Ansätzen einen Platz (54, 74) zur Aufnahme eines Gegenstands (2) aufweist, derart dass dieser Gegenstand in dem genannten Aufnahmeplatz (54, 74) bei einem eine Rotation dieses Aufnahmepkatzes hervorrufenden Drehantrieb des Drehkreuzes bzw. Drehtellers (8, 24) verschoben bzw. transportiert werden kann, **dadurch gekennzeichnet, dass** die Anordnung des weiteren ein erstes Überführungsmittel (18) umfasst, welches eine Transportverschiebung eines Gegenstands (2), welcher sich in einem Aufnahmeplatz (54) des Eingangs-Transport- Drehkreuzes bzw. -Drehtellers (8) befindet, der mit einem Eingang
(60) der genannten Behandlungskammer (14) in Verbindung steht, bis ins Innere dieser Behandlungskammer (14) gestattet;
dass die Anordnung des weiteren ein zweites Überführungsmittel (22) umfasst, welches die Transportverschiebung eines im Inneren der Behandlungskammer (14) befindlichen Gegenstands (2) bis in eien Aufnahmeplatz (24) des Ausgangs-Transport- Drehkreuzes bzw. -Drehtellers (24) gestattet, welcher mit einem Ausgang (66) dieser Behandlungskammer (14) in Verbindung steht;
dass die genannte Behandlungskkammer (14) mit wenigstens einem inneren Förderer bzw. Konveyer (16, 20) versehen ist, welcher die Gegenstände (2) im Inneren der Kammer (14) in Bewegung zu setzen gestattet, wobei diese inneren Förderer einen internen Primärförderer (16), auf welchen die Gegenstände (2) mittels des genannten zweiten Überführungsmittels (18) zugeführt werden können, sowie einen internen Sekundärförderer (20) umfassen, von welchem die Gegenstände (2) in einen Aufnahmeplatz (74) des Ausgangs-Transport-Drehkreuzes bzw. -Drehtelllers (24) verbracht werden können vermittels des genannten zweiten Überführungsmittels (28);
dass ein stromabwärtiges Ende (16 b) des internen Primärförderrers (16) in Ausrichtung mit und in Abstand von einem stromaufwärtigen Ende (20 b) des internen Sekundärförderers (20) angeordnet ist, zur Bildung eines Zwischenraums (68) zwischen Förderern; und
dass die genannten Sterilisiermittel (34) eine Mehrzahl von Quellen (80a, 80b, 80c) umfassen, deren jede jeweils ein Elektronenstrahlbündel entlang einer Achse (A1, A2, A3) erzeugen, die jeweils durch den Raum (68) zwischen den Förderern verläuft.

2. Anordnung (1) für die Sterilisierung von Gegenständen, nach Anspruch 1, **dadurch gekennzeichnet, dass** sie ein drittes Überführungsmittel (12) umfasst, welches die Transportverschiebung eines am stromabwärtigen Ende (6a) des ersten Förderers bzw. Konveyers (6) befindlichen Gegenstands (2) bis in einen Aufnahmeplatz (54) des Bingangs-Transport-Drekreuzes bzw. -Drehtellers (8) gestattet, der bzw. das in Ausrichtung mit diesem stromabwärtigen Ende (6a) des ersten Förderers (6) angeordnet ist.

3. Anordnung (1) zur Sterilisierung von Gegenständen, nach Anspruch 1 oder Anspruch 2, **dadurch gekennzeichnet, daß** die Anordnung ein viertes Überführungsmittel (28) umfasst, welches die Transportverschiebung eines Gegenstands (2), der sich in einem Aufnahmeplatz (74) des Ausgangs-Transport-Drehkreuzes bzw. -Drehtellers (24) in Ausrichtung mit einem stromaufwärtigen Ende (30a) des zweiten Förderers (30) befindet, bis auf dieses stromaufwärtige Ende des zweiten Förderers (30) gestattet.

4. Anordnung (1) für die Sterilisierung von Gegenständen, nach einem der vorhergehenden Ansprüche, **dadurch gekennzeichnet dass** der genannte interne Primär-Förderer bzw.-Konveyer (16) ein benachbart dem Eingang (60) der Behandlungskammer (14) befindliches strom-aufwärtiges Ende (16a) aufweist, und dass der genannte interne Sekundär-Förderer bzw. -Konveyer (20) ein benachbart dem Ausgang (66) der Behandlungskammer (14) befindliches stromabwärtiges Ende (20a) aufweist.

5. Anordnung (1) für die Sterilisierung von Gegenständen, nach einem beliebigen der vorhergehenden Ansprüche, **dadurch gekennzeichnet, dass** die genannten internen Primär- (16) und Sekundär-Förderer (20) alle im wesentlichen geradlinig und im wesentlichen entlang ein und derselben Geraden parallel zu einer Vorschubrichtung (48) der Gegenstände sind.

6. Anordnung (1) für die Sterilisierung von Gegenständen, nach einem beliebigen der vorhergehenden Ansprüche, **dadurch gekennzeichnet, dass** die genannten Sterilisiermittel (34) drei Quellen (80a, 80b, 80c) umfassen, welche entsprechend drei Elektronenstrahlbündel entlang Achsen (A1, A2, A3) erzeugen, welche die Behandlungskammer (14) durchsetzen, wobei diese Achsen jeweils unter einem Winkel von 120° relativ zueinander in einer zu der Vorschubrichtung der Gegenstände (48) rechrwinkligen Ebene P angeordnet sind.

7. Anordnung (1) für die Sterilisierung von Gegenständen, nach Anspruch 6, **dadurch gekennzeichnet, dass** die genannte Ebene P den genannten Raum (68) zwischen den Förderern durchsetzt.

8. Anordnung (1) für die Sterilisierung von Gegenständen, nach einem beliebigen der vorhergehenden Ansprüche, **dadurch gekennzeichnet, dass** der genannte zweite Förderer bzw. Konveyer (30) in einem Produkt-Sammler (32) mündet, in den die sterilisierten Gegenstände zugeführt werden.

9. Anlage (1) für die Sterilisierung von Gegenständen nach einem beliebigen der vorhergehenden Ansprüche, **dadurch gekennzeichnet, dass** das genannte Eingangs-Transport-Drehkreuz bzw. Drehteller (8) in einer Krone (10) angeordnet ist, welche einerseits eine Öffnung (50) in Ausrichtung mit dem ersten Förderer bzw. Konveyer (6) aufweist, um die Einführung der Gegenstände (2) in das genannte Eingangs-Transport-Drehkreuz (8) zu gewährleisten, und andererseits eine Öffnung (58) aufweist, die in der Verlängerung und stromaufwärtig von dem Eingang (60) der genannten Behandlungskammer (14) gelegen ist, und dass das Ausgangs-Transport-Drehkreuz (24) in einer Krone (26) angeordnet ist, welche einerseits eine in der Verlängerung und stromaufwärtig des Ausgangs (66) der Behandlungskammer (14) gelegene Öffnung (70) aufweist und andererseits eine Öffnung (78) in Ausrichtung mit dem zweiten Förderer bzw. Konveyer (30), um die Entnahme der Gegenstände (2) aus dem Ausgangs-Transport-Drehkreuz (24) gewährleisten zu können.
